# EUROPEAN PATENT APPLICATION

(11) **EP 4 434 971 A1**
(43) Date of publication of application: **25.09.2024**
(21) Application number: 22895626.4
(22) Date of filing: 16.11.2022
(51) Int. Cl.: C07D 233/64, C07B 61/00, B01J 31/02

(54) **METHOD FOR PRODUCING 2-ALKYLTHIO-1-IMIDAZOYLETHANONE COMPOUND**

(30) Priority: 19.11.2021 JP 2021188983
(71) Applicant: Nippon Soda Co., Ltd., Tokyo 100-7010 (JP)
(72) Inventor: YASUDA, Kohei, Takaoka-shi, Toyama 933-8507 (JP); HAKUTA, Hiroshi, Takaoka-shi, Toyama 933-8507 (JP)
(74) Representative: Sonnenhauser, Thomas Martin
(86) International application number: PCT/JP2022/042500
(87) International publication number: WO 2023/090345

(57) **Abstract**

Provided is a method for producing a compound represented by formula (3) (wherein R¹, R², R³ and R^{b} are the same as those in formulae (1) and (2)), the method including chemically reacting a compound represented by formula (1) (wherein R¹ represents a C1-C6 alkyl group, R² represents a hydrogen atom, a halogeno group, an (un)substituted C1-C6 alkyl group, an (un)substituted C2-C6 alkenyl group, or a 1,3-dioxolan-2-yl group, and R^{a} represents a C1-C6 alkyl group) with a compound represented by formula (2) (wherein R³ represents a C1-C6 alkyl group, and R^{b} represents a C1-C6 alkyl group) in the presence of a Lewis acid and a base.

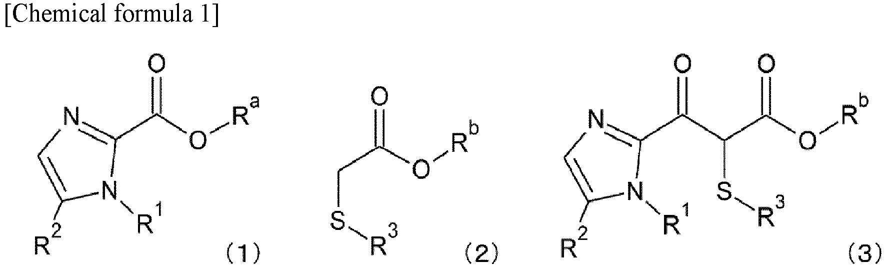

## Description

### [Technical Field]

The present invention relates to a method of producing a 2-alkylthio-1-imidazoyl ethanone compound.

Priority is claimed on Japanese Patent Application No. 2021-188983, filed November 19, 2021, the content of which is incorporated herein by reference.

### [Background Art]

Nitrogen-containing heterocycles are main partial structures (building blocks) that constitute compounds that serve as raw materials of pharmaceuticals or agricultural chemicals. In recent years, the development of harmful arthropod control agents having an imidazole ring, a pyridine ring or the like has been actively carried out. For example, Patent Document 1 discloses an ethylsulfonyl-substituted imidazoylpyrimidine compound, and describes a compound of formula (A) as an intermediate.

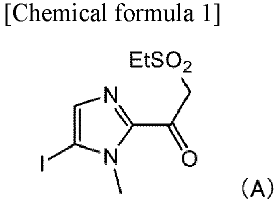

Patent Document 1 discloses that the compound of formula (A) is produced by the following steps.

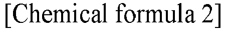

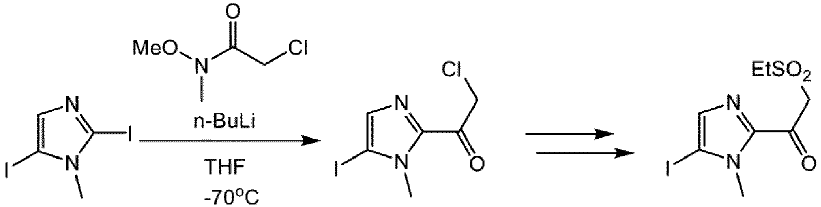

In addition, Patent Document 2 discloses that an imidazole compound is produced by the following step.

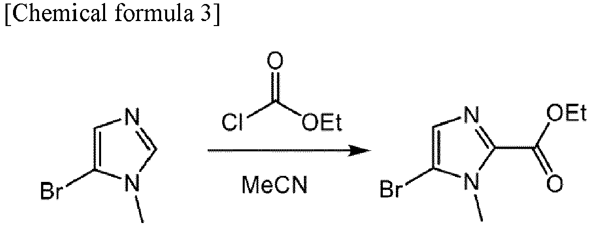

In addition, Patent Document 3 discloses that an imidazole compound is produced by the following step.

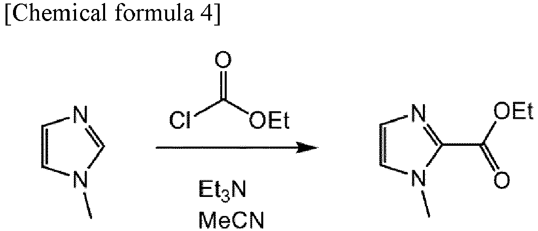

### [Citation List]

### [Patent Document]

[Patent Document 1]
   WO 2020/071304 A
[Patent Document 2]
   WO 2005/090333 A
[Patent Document 3]
   Japanese Unexamined Patent Application Publication No. 2017-66077 A

### [Summary of Invention]

### [Technical Problem]

An object of the present invention is to provide a method of producing a 2-alkylthio-1-imidazoyl ethanone compound, which is one of building blocks. An additional object of the present invention is to provide an imidazoyl ethanone compound which serves as a raw material thereof.

### [Solution to Problem]

The present invention includes the following aspects and was completed as a result of repeated studies to achieve the above-mentioned objects.
(1) A method of producing a compound of formula (3), including: chemically reacting a compound of formula (1): 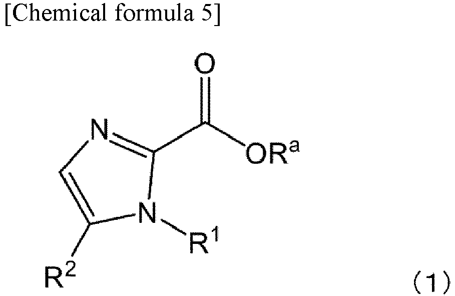 (in the formula (1), R¹ is a C1-6 alkyl group, R² is a hydrogen atom, a halogeno group, a substituted or unsubstituted C1-6 alkyl group, a substituted or unsubstituted C2-6 alkenyl group, or a 1,3-dioxolan-2-yl group, and R^{a} is a C1-6 alkyl group), and a compound of formula (2): 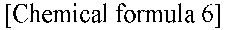 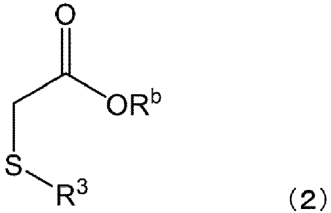 (in the formula (2), R³ is a C1-6 alkyl group and R^{b} is a C1-6 alkyl group) in the presence of a Lewis acid and a base, 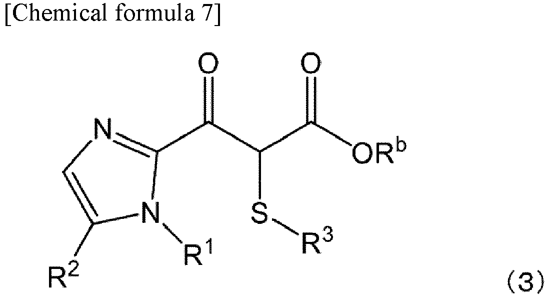 (in the formula (3), R¹, R², R³, and R^{b} are identical to R¹, R², R³, and R^{b} in the formula (1) and the formula (2), respectively).
(2) The production method according to (1) mentioned above, wherein the Lewis acid is a Ti compound of formula (4):

   [Chemical formula 8]

   Ti(X)ₘ(OR^{c})ₙ (4)

   (in the formula (4), X is a halogeno group, R^{c} is a C1-6 alkyl group, m is an integer of 0 to 4, n is an integer of 0 to 4, and m+n=4).
(3) A compound of formula (3a): 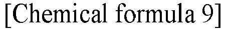 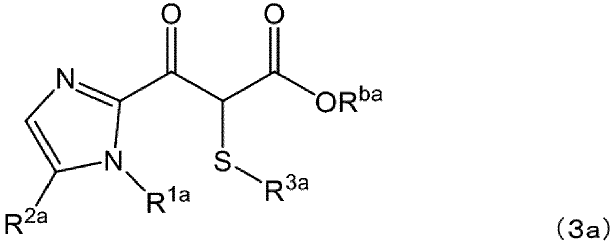 (in the formula (3a), R^{1a} is a C1-6 alkyl group, R^{2a} is a C2-6 haloalkenyl group, R^{3a} is a C1-6 alkyl group, and R^{ba} is a C1-6 alkyl group).
(4) A compound of formula (1a): 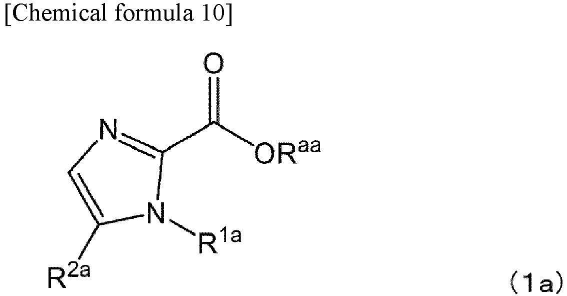 (in the formula (1a), R^{1a} is a C1-6 alkyl group, R^{2a} is a C2-6 haloalkenyl group, and R^{aa} is a C1-6 alkyl group).
(5) A method of producing a compound of formula (1a), including:
   an addition step in which a chloroformate of formula: ClCO₂R^{aa} (in the formula, R^{aa} is a C1-6 alkyl group) is added to a liquid mixture of a compound of formula (7), diisopropylethylamine and chloroform, 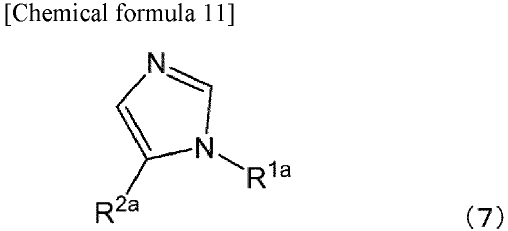 (in the formula (7), R^{1a} is a C1-6 alkyl group, and R^{2a} is a C2-6 haloalkenyl group), and
   a reaction step in which the compound of formula (7) and the chloroformate are reacted in the presence of the diisopropylethylamine to obtain the compound of formula (1a): 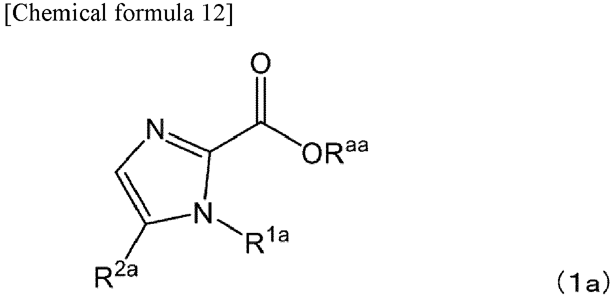 (in the formula (1a), R^{1a} is a C1-6 alkyl group, R^{2a} is a C2-6 haloalkenyl group, and R^{aa} is a C1-6 alkyl group).

### [Advantageous Effects of Invention]

According to the production method of the present invention, the compound of formula (3) can be obtained in a high yield. In addition, the compound of formula (1), particularly the compound of formula (1a), which serves as a raw material thereof, can be obtained in a high yield.

### [Description of Embodiments]

In the present invention, the term "unsubstituted" refers to a group consisting of a mother nucleus. In the case where only the name of a group serving as a mother nucleus is provided without accompanying the term "substituted", this refers to "unsubstituted" unless specifically indicated otherwise.

On the other hand, the term "substituted" means that any hydrogen atom of a group serving as a mother nucleus is substituted with a group (substituent) having a structure that is identical to or different from the mother nucleus. Thus, a "substituent" is another group bound to a group serving as the mother nucleus. The number of substituents may be one or two or more. Two or more substituents may be identical to or different from each other.

The term "C1-6", for example, indicates that the number of carbon atoms of the group serving as the mother nucleus is 1 to 6. The number of carbon atoms does not include the number of carbon atoms present in substituents. For example, a butyl group having an ethoxy group as a substituent thereof is classified as a C2 alkoxy C4 alkyl group.

There are no particular limitations on "substituents" provided that they are chemically acceptable and achieve the effects of the present invention.

Examples of groups that can be "substituents" include the following groups:
C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group;
C2-6 alkenyl groups such as a vinyl group, a 1-propenyl group, a 2-propenyl group (an allyl group), a 1-butenyl group, a 2-butenyl group, a 3-butenyl group, a 1-methyl-2-propenyl group, and a 2-methyl-2-propenyl group;
C2-6 alkynyl groups such as an ethynyl group, a 1-propynyl group, a 2-propynyl group, a 1-butynyl group, a 2-butynyl group, a 3-butynyl group, and a 1-methyl-2-propynyl group;
C3-6 cycloalkyl groups such as a cyclopropyl group, a cyclobutyl group, a cyclopentyl group, and a cyclohexyl group;
a phenyl group;
three- to six-membered heterocyclyl groups;
C1-6 alkoxy groups such as a methoxy group, an ethoxy group, a n-propoxy group, an i-propoxy group, a n-butoxy group, a s-butoxy group, an i-butoxy group, and a t-butoxy group;
C6-10 aryloxy groups such as a phenoxy group, and a naphthoxy group;
five- or six-membered heteroaryloxy groups such as a thiazolyloxy group, and a pyridyloxy group;
C1-6 alkoxycarbonyl groups such as a methoxycarbonyl group, an ethoxycarbonyl group, a n-propoxycarbonyl group, an i-propoxycarbonyl group, a n-butoxycarbonyl group, and a t-butoxycarbonyl group;
halogeno groups such as a fluoro group, a chloro group, a bromo group, and an iodo group;
C1-6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group;
C1-6 haloalkoxy groups such as a trifluoromethoxy group, a 2-chloro-n-propoxy group, and a 2,3-dichlorobutoxy group;
a formylamino group;
C1-6 alkylcarbonylamino groups such as an acetylamino group, a propanoylamino group, a butyrylamino group, and an i-propylcarbonylamino group;
C1-6 alkoxycarbonylamino groups such as a methoxycarbonylamino group, an ethoxycarbonylamino group, a n-propoxycarbonylamino group, and an i-propoxycarbonylamino group;
unsubstituted or substituted aminocarbonyl groups such as an aminocarbonyl group, a dimethylaminocarbonyl group, a phenylaminocarbonyl group, and a N-phenyl-N-methylaminocarbonyl group;
C1-6 alkylthio groups such as a methylthio group, an ethylthio group, a n-propylthio group, an i-propylthio group, a n-butylthio group, an i-butylthio group, a s-butylthio group, and a t-butylthio group;
C1-6 haloalkylthio groups such as a trifluoromethylthio group, and a 2,2,2-trifluoroethylthio group;
C1-6 alkylsulfonyl groups such as a methylsulfonyl group, an ethylsulfonyl group, and a t-butylsulfonyl group;
C1-6 haloalkylsulfonyl groups such as a trifluoromethylsulfonyl group, and a 2,2,2-trifluoroethylsulfonyl group;
a cyano group; and a nitro group.

Any hydrogen atom of the "substituent" may be substituted with a group having a different structure. Examples of such a substituent include C1-6 alkyl groups, C1-6 haloalkyl groups, C1-6 alkoxy groups, C1-6 haloalkoxy groups, halogeno groups, a cyano group, and a nitro group.

The "three- to six-membered heterocyclyl group" is one which contains, as a ring member atom, one to four hetero atoms selected from the group consisting of a nitrogen atom, an oxygen atom and a sulfur atom. The heterocyclyl group may be monocyclic or polycyclic. If at least one ring of a polycyclic heterocyclyl group is a hetero ring, the remaining rings thereof may be any of saturated alicyclic rings, unsaturated alicyclic rings and aromatic rings. Examples of the "three- to six-membered heterocyclyl group" include three- to six-membered saturated heterocyclyl groups, five- or six-membered heteroaryl groups, and five- or six-membered partially unsaturated heterocyclyl groups.

Examples of the three- to six-membered saturated heterocyclyl groups include an aziridinyl group, an epoxy group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the five-membered heteroaryl groups include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the six-membered heteroaryl groups include a pyridyl group, a pyrazinyl group, a pyrimidinyl group, a pyridazinyl group, and a triazinyl group.

Examples of the five-membered partially-unsaturated heterocyclyl groups include a pyrrolinyl group, a dihydrofuranyl group, an imidazolinyl group, a pyrazolinyl group, an oxazolinyl group, and an isooxazolinyl group.

Examples of the six-membered partially-unsaturated heterocyclyl groups include a dihydropyranyl group.

A method of producing a compound (3) of the present invention includes chemically reacting a compound (1) and a compound (2) in the presence of a Lewis acid and a base.

The compound (1) is represented by the following formula (1).

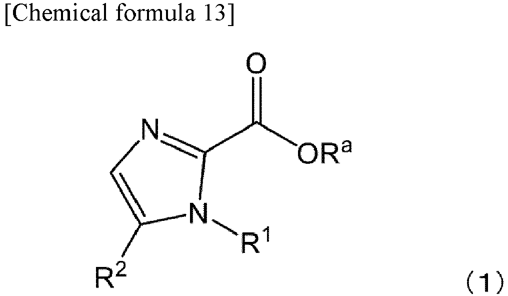

In the formula (1), R¹ is a C1-6 alkyl group.

The C1-6 alkyl group as R¹ may be linear or branched. Examples of the C1-6 alkyl group as R¹ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, a 2,2-dimethylpropyl group, and an i-hexyl group,
In the formula (1), R² is a hydrogen atom, a halogeno group, a substituted or unsubstituted C1-6 alkyl group, a substituted or unsubstituted C2-6 alkenyl group, or a 1,3-dioxolan-2-yl group.

Examples of the "halogeno group" as R² include a fluoro group, a chloro group, a bromo group, and an iodo group.

Examples of the "C1-6 alkyl group" as R² include the same groups as those mentioned as R¹.

Examples of the "C2-6 alkenyl group" as R² include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

Examples of a substituent on the "C1-6 alkyl group" or "C2-6 alkenyl group" as R² include: halogeno groups such a fluoro group, a chloro group, a bromo group, and an iodo group; a hydroxy group; C1-6 alkoxy groups such as a methoxy group, an ethoxy group, a n-propoxy group, an i-propoxy group, a n-butoxy group, a s-butoxy group, an i-butoxy group, and a t-butoxy group; C1-6 haloalkoxy groups such as a 2-chloro-n-propoxy group, a 2,3-dichlorobutoxy group, and a trifluoromethoxy group; a phenyl group; phenyl groups substituted with a halogeno group, a C1-6 haloalkyl group, or a C1-6 haloalkoxy group, such as a 4-chlorophenyl group, a 4-trifluoromethylphenyl group, and a 4-trifluoromethoxyphenyl group; and a cyano group. Among these, halogeno groups are preferable.

Examples of the "halogeno-substituted C1-6 alkyl group (which may be referred to as a C1-6 haloalkyl group)" as R² include a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a 1-chloro-2,2,3,3,3-pentafluoropropyl group, a 1,2,2,3,3,3-hexafluoropropyl group, a perfluoropropyl group, a 2,2,2-trifluoro-1-trifluoromethylethyl group, a perfluoro isopropyl group, a 4-fluorobutyl group, a 1,4,4,4-tetrafluorobutyl group, a 2,2,3,3,4,4,4-heptafluorobutyl group, a 1,2,2,3,3,4,4,4-octafluorobutyl group, a perfluorobutyl group, a 3,3,3-trifluoro-2-trifluoromethylpropyl group, a 1-chloro-2,3,3,3-tetrafluoro-2-trifluoromethylpropyl group, a 1,2,3,3,3-pentafluoro-2-trifluoromethylpropyl group, a perfluoropentyl group, and a perfluorohexyl group.

Examples of the "halogeno-substituted C2-6 alkenyl group (which may be referred to as a C2-6 haloalkenyl group)" as R² include a 2,3,3,3-tetrafluoro-1-propenyl group, a 3,3,3-trifluoro-1-propenyl group, a 2-chloro-3,3,3-trifluoro-1-propenyl group, a 2-bromo-3,3,3-trifluoro-1-propenyl group, a 3,3,3-trifluoro-2-trifluoromethyl-1-propenyl group, a 3,3,4,4,4-pentafluoro-1-butenyl group, a 2,3,3,4,4,4-hexafluoro-1-butenyl group, and a 2-chloro-3,3,4,4,4-pentafluoro-1-butenyl group.

In the formula (1), R^{a} is a C1-6 alkyl group.

Examples of the "C1-6 alkyl group" as R^{a} include the same groups as those mentioned as R¹.

The compound (1) is preferably a compound of the following formula (1a) (hereinafter, may be referred to as compound (1a)).

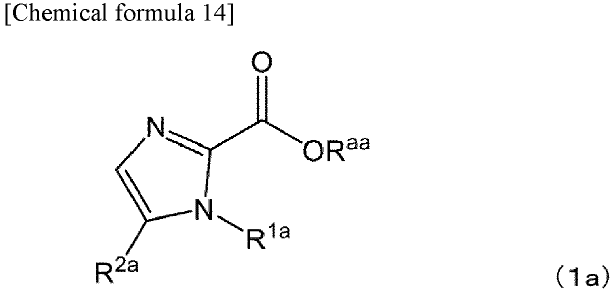

In the formula (1a), R^{1a} is a C1-6 alkyl group, R^{2a} is a C2-6 haloalkenyl group, and R^{aa} is a C1-6 alkyl group.

The compound (1a) is a novel compound and is useful as a substrate in the production method according to the present invention.

Examples of the "C1-6 alkyl group" as R^{1a} include the same groups as those mentioned as R¹, and a methyl group is preferable.

Examples of the "C1-6 alkyl group" as R^{aa} include the same groups as those mentioned as R^{a}, and an ethyl group is preferable.

Examples of the "C2-6 haloalkenyl group" as R^{2a} include the same groups as those mentioned as R², and a 2-chloro-3,3,3-trifluoro-1-propenyl group is preferable. The 2-chloro-3,3,3-trifluoro-1-propenyl group may be a mixture of E/Z, a single Z, or a single E, in terms of stereoisomerism.

The compound (1) used in the present invention may be one which is synthesized using a conventionally-known method, or one which is synthesized by any method and commercially available. Specific examples of the compound (1) include the following compounds.

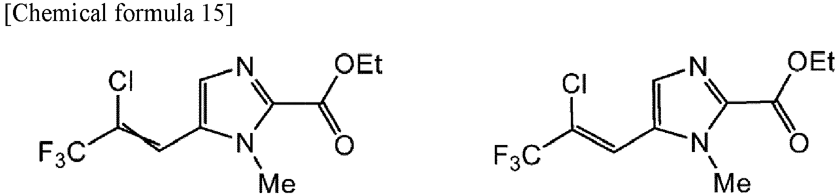

The following notation in the above chemical formula indicates a double bond whose steric configuration is not defined (undefined double stereo bond).

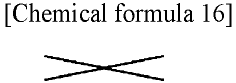

The compound (2) is represented by the following formula (2).

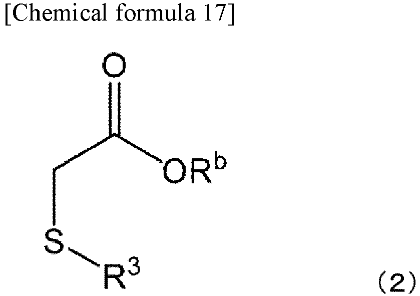

In formula (2), R³ is a C1-6 alkyl group and R^{b} is a C1-6 alkyl group.

Examples of the C1-6 alkyl group as R³ or R^{b} include the same groups as those mentioned as R^{a}, and an ethyl group is preferable.

The compound (2) used in the present invention may be one which is synthesized using a conventionally-known method, or one which is synthesized by any method and commercially available.

The amount of the compound (2) used relative to one mol of the compound (1) is, for example, 1.0 mol to 5.0 mol, and preferably 1.1 mol to 2.0 mol.

The Lewis acid in the above-mentioned chemical reaction is composed of a central metal element and electron-withdrawing groups that bind to the metal.

Examples of the central metal element include boron (B), aluminum (Al), scandium (Sc), titanium (Ti), iron (Fe), zinc (Zn), arsenic (As), zirconium (Zr), niobium (Nb), cadmium (Cd), indium (In), tin (Sn), antimony (Sb), hafnium (Hf), mercury (Hg), and lanthanides. Among these, titanium (Ti) is preferable.

Examples of the electron-withdrawing groups include halogeno groups and triflate groups.

In the present invention, a Ti compound of the following formula (4) (hereinafter, may be referred to as compound (4)) can be preferably used as the Lewis acid.
[Chemical formula 18]

Ti(X)ₘ(OR^{c})ₙ (4)

In the formula (4), X is a halogeno group, R^{c} is a C1-6 alkyl group, m is an integer of 0 to 4, n is an integer of 0 to 4, and m+n=4.

Examples of the "halogeno group" as X include the same groups as those mentioned as R².

Examples of the "C1-6 alkyl group" as R^{c} include the same groups as those mentioned as R¹.

The compound (4) which is commercially available may be used, or the compound (4) may be prepared by mixing tetrahalogeno titanium (TiX₄) and alcohol (R^{c}OH) in the chemical reaction site.

Examples of the compound (4) preferably used in the present invention include TiCl₃(OⁱPr). ⁱPr is an isopropyl group.

The amount of the Lewis acid used relative to one mol of the compound (1) is, for example, 1.0 mol to 5.0 mol, and preferably 1.1 mol to 4.0 mol.

Examples of the base in the chemical reaction include: inorganic bases such as sodium carbonate, sodium hydrogen carbonate, potassium carbonate, potassium hydrogen carbonate, sodium hydroxide, potassium hydroxide, sodium hydride, and potassium hydride; and organic bases such as triethylamine, tetramethylethylenediamine, N,N-diisopropylamine, N,N-dimethylaniline, N,N-diethylaniline, 4-methylmorpholine, 1-azabicyclo[2.2.2]octane, 1,4-diazabicyclo[2.2.2] octane (abbreviation: DABCO), 1,8-diazabicyclo[5.4.0]undec-7-ene, 1,5-diazabicyclo[4.3.0]non-5-ene, pyridine, 4-(dimethylamino)pyridine and 2,6-dimethylpyridine, and organic bases such as triethylamine are preferably used.

The amount of the base used relative to one mole of the Lewis acid is, for example, 1.0 mol to 5.0 mol, and preferably 1.0 mol to 3.0 mol.

The chemical reaction may be carried out in the absence of a solvent or in a solvent. The chemical reaction is preferably carried out in a solvent in view of operability.

The organic solvent is not particularly limited as long as the organic solvent is inert to the Lewis acid. Examples of the organic solvent include: ethers such as diethyl ether, diisopropyl ether, diethylene glycol dimethyl ether (product name: diglyme) and tetrahydrofuran (abbreviation: THF); halogenated hydrocarbons such as dichloromethane, chloroform, and 1,2-dichloroethane (abbreviation: DCE); aliphatic hydrocarbons such as pentane, hexane, cyclohexane, heptane and octane; aromatic hydrocarbons such as toluene and xylene; aprotic polar solvents such as N,N- dimethylformamide (abbreviation: DMF), N,N'-dimethylpropyleneurea (abbreviation: DMPU) and hexamethylphosphoric triamide (abbreviation: HMPA); and acetonitrile, and halogenated hydrocarbons such as chloroform are preferably used.

The amount of the organic solvent used relative to one part by weight of the compound (1) is preferably 5 parts by weight to 500 parts by weight.

The order of addition of the compound (1), the compound (2), the base, and the Lewis acid used in the chemical reaction, and the organic solvent used as necessary, to the reaction site is not particularly limited.

One embodiment includes: adding a Lewis acid to an organic solvent or in the absence of a solvent; adding the compound (2) thereto, and then adding the compound (1) thereto to obtain a liquid mixture, followed by adding a base to the liquid mixture while stirring to terminate the chemical reaction.

Another embodiment includes: adding dropwise tetrahalogeno titanium and alcohol in an organic solvent to prepare a Lewis acid, adding dropwise the compound (2) thereto, further adding dropwise the compound (1) thereto to obtain a liquid mixture, adding dropwise a base to the liquid mixture while stirring to carry out a chemical reaction, adding an organic solvent to a cooled reaction liquid, adding dropwise tetrahalogeno titanium and alcohol thereto, and further adding dropwise a base while stirring to carry out the chemical reaction. The dropwise addition is conducted while stirring.

Although each substance may be added all at once or gradually, it is preferable that dropwise addition be conducted to add a small amount thereof gradually, and it is more preferable that dropwise addition be conducted while stirring.

Although the temperature at which each substance is added (added dropwise) is not particularly limited, the temperature is preferably 0°C.

The temperature in the chemical reaction is not particularly limited, and is, for example, 0°C to 60°C, and preferably 30°C to 50°C.

The pressure in the chemical reaction is, for example, 0.1 MPa to 1 MPa, preferably 0.1 MPa to 0.5 MPa, and more preferably 0.1 MPa.

The reaction time in the chemical reaction is not particularly limited, and is, for example, 0.5 hours to 24 hours.

The chemical reaction is preferably carried out under an inert gas atmosphere.

After the chemical reaction is completed, extraction may be conducted with an organic solvent, and the resultant organic layer may be subjected to a post-treatment such as drying and concentrating. Furthermore, if necessary, the resultant in the chemical reaction may be purified by an operation such as recrystallization or chromatography.

The compound (3) obtained by the production method of the present invention is represented by the following formula (3).

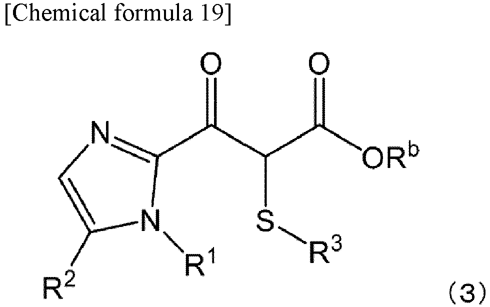

In the formula (3), R¹, R², R³, and R^{b} are identical to R¹, R², R³, and R^{b} in the formula (1) and the formula (2), respectively.

Among the compounds (3) obtained by the production method according to the present invention, a compound of the following formula (3a) (hereinafter, may be referred to as compound (3a)) is a novel compound and is particularly useful as an intermediate of a pharmaceutical or agricultural chemical.

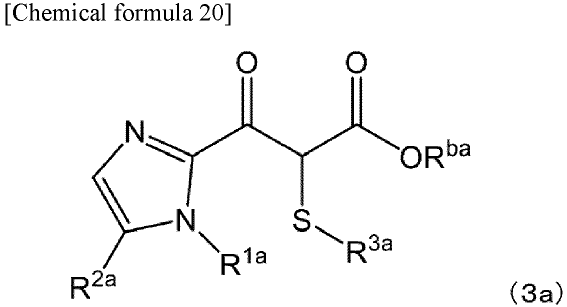

In the formula (3a), R^{1a} is a C1-6 alkyl group, and preferably a methyl group.

R^{2a} is a C2-6 haloalkenyl group, and preferably a 2-chloro-3,3,3-trifluoro-1-propenyl group.

R^{3a} is a C1-6 alkyl group, and preferably an ethyl group.

R^{ba} is a C1-6 alkyl group, and preferably an ethyl group.

Specific examples of the compound (3a) include the following compounds.

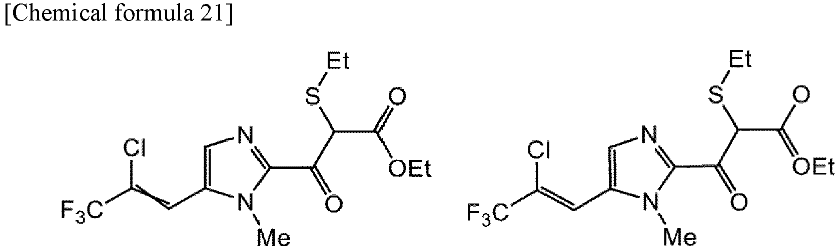

The compound (3) can be converted to a compound of the following formula (5) (hereinafter, may be referred to as compound (5)) by a decarboxylation reaction.

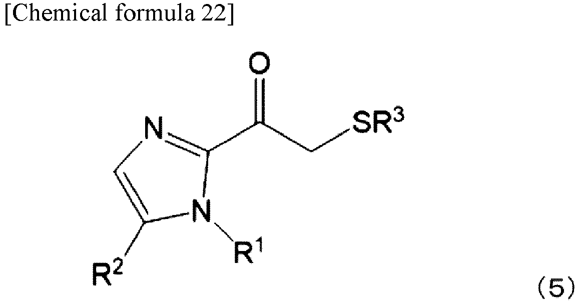

In the formula (5), R¹, R², and R³ are the same groups as those in the formula (3), respectively.

Among them, R¹ is preferably a methyl group. R² is preferably a 2-chloro-3,3,3-trifluoro-1-propenyl group. R³ is preferably an ethyl group.

Specific examples of the compound (5) include the following compounds.

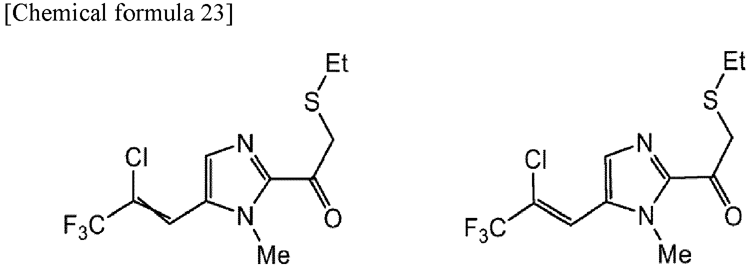

The decarboxylation reaction may be conducted by a conventionally-known method. Examples thereof include a method of performing decarboxylation in the presence of an acid.

Examples of the acid used in the decarboxylation reaction include carboxylic acids such as acetic acid, formic acid, oxalic acid, benzoic acid, and 4-chlorobenzoic acid; phosphoric acid; sulfonic acids such as methanesulfonic acid, 4-toluenesulfonic acid monohydrate, and trifluoromethanesulfonic acid; hydrogen halides such as hydrogen chloride, hydrogen bromide, hydrogen iodide and hydrogen fluoride; sulfuric acid, nitric acid, and tetrafluoroboric acid.

The amount of the acid used relative to one mol of the compound (3) is, for example, 0.5 mol to 3.0 mol, and preferably 1.0 mol to 2.0 mol.

The decarboxylation reaction is preferably carried out in a solvent. For example, the decarboxylation reaction may be carried out by adding dropwise the acid to a liquid mixture of the compound (3) and a solvent while stirring at room temperature, followed by heating the resultant while continuing stirring.

Examples of a solvent which may be used include water, organic solvents, mixed solvents of water and organic solvents, and mixed solvents of organic solvents.

Examples of the organic solvents include alcohols such as methanol and ethanol; ethers such as diethyl ether, diisopropyl ether, diethylene glycol dimethyl ether (product name: diglyme) and tetrahydrofuran (abbreviation: THF); esters such as ethyl acetate; halogenated hydrocarbons such as dichloromethane, chloroform and 1,2-dichloroethane (abbreviation: DCE); aliphatic hydrocarbons such as pentane, hexane, cyclohexane, heptane and octane; aromatic hydrocarbons such as toluene and xylene; aprotic polar solvents such as N,N-dimethylformamide (abbreviation: DMF), N,N'-dimethylpropylene urea (abbreviation: DMPU), and hexamethylphosphoric triamide (abbreviation: HMPA); and acetonitrile.

The amount of the solvent used relative to one part by weight of the compound (3) is preferably 5 parts by weight to 500 parts by weight.

The decarboxylation reaction is preferably carried out while heating, for example, at a temperature of 40°C to 110°C, although the temperature depends on the boiling point of the solvent used.

The reaction time in the decarboxylation reaction is not particularly limited, and is, for example, 0.5 hours to 24 hours.

The compound (5) can be converted to a compound of the following formula (6) (hereinafter, may be referred to as compound (6)) by an oxidation reaction.

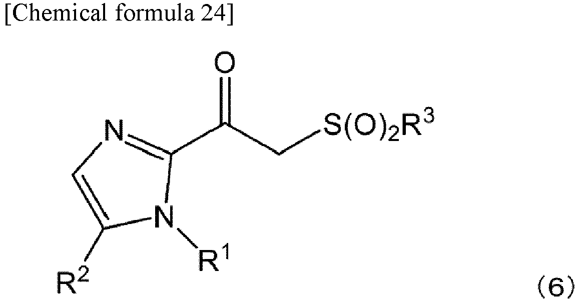

In the formula (6), R¹, R² and R³ are the same as those in the formula (5), respectively.

Specific examples of the compound (6) include the following compounds.

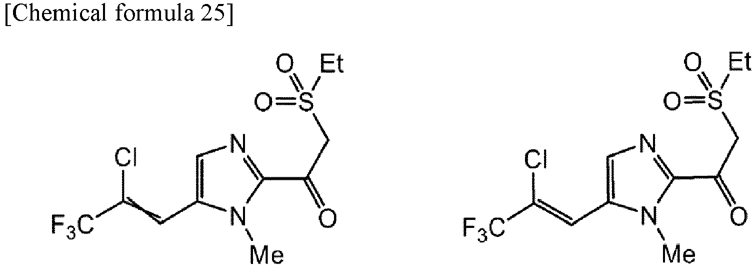

The production method of the compound (1a) includes: an addition step in which a chloroformate of formula: ClCO₂R^{aa} (in the formula, R^{aa} is a C1-6 alkyl group) is added to a liquid mixture composed of a compound of the following formula (7) (hereinafter, may be referred to as compound (7)), diisopropylethylamine and chloroform; and a reaction step in which the compound (7) and the chloroformate are reacted in the presence of the diisopropylethylamine to obtain the compound (1a).

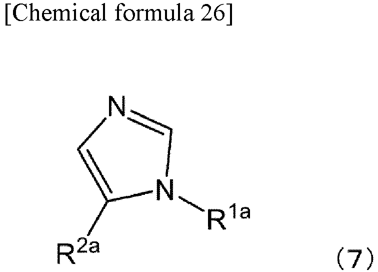

In the formula (7), R^{1a} is a C1-6 alkyl group, and R^{2a} is a C2-6 haloalkenyl group.

In the formula (7), R^{1a} is the same as R^{1a} in the formula (1), and is preferably a methyl group.

In the formula (7), R^{2a} is the same as R^{2a} in the formula (1), and is preferably a 2-chloro-3,3,3-trifluoro-1-propenyl group.

Specific examples of the compound (7) include the following compounds.

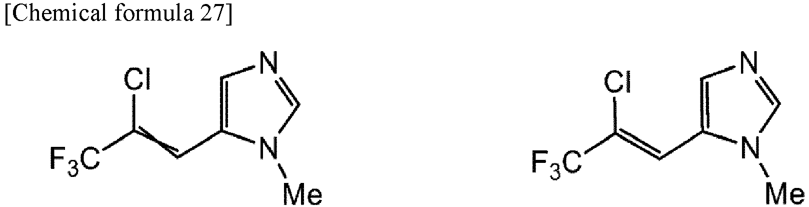

The amount of the diisopropylethylamine used relative to one mol of the compound (7) is, for example, 0.9 mol to 3.0 mol, and preferably 1.0 mol to 2.5 mol.

The amount of the chloroform used relative to one part by weight of the compound (7) is preferably 5 parts by weight to 500 parts by weight.

In the addition step, another organic solvent may be further added to the liquid mixture to obtain a mixed solvent, and the reaction step may be carried out in the mixed solvent, in view of operability.

The organic solvent is not particularly limited as long as the organic solvent is inert to the chloroformate. Examples of the organic solvent include: ethers such as diethyl ether, diisopropyl ether, diethylene glycol dimethyl ether (product name: diglyme) and tetrahydrofuran (abbreviation: THF); halogenated hydrocarbons such as dichloromethane and 1,2-dichloroethane (abbreviation: DCE); aliphatic hydrocarbons such as pentane, hexane, cyclohexane, heptane and octane; aromatic hydrocarbons such as toluene and xylene; aprotic polar solvents such as N,N-dimethylformamide (abbreviation: DMF), N,N'-dimethylpropylene urea (abbreviation: DMPU), hexamethylphosphoric triamide (abbreviation: HMPA); and acetonitrile.

Specific examples of the chloroformate of the formula: ClCO₂R^{aa} include methyl chloroformate, ethyl chloroformate, and n-propyl chloroformate. An ethyl chloroformate is particularly preferable.

The amount of the chloroformate used relative to one mol of the compound (7) is, for example, 0.9 mol to 3.0 mol, and preferably 1.0 mol to 2.5 mol.

Although the chloroformate may be added all at once or gradually, it is preferably added dropwise gradually, and more preferably added dropwise while stirring.

Although the temperature at which the chloroformate is added is not particularly limited, the temperature is room temperature to 45°C, and preferably 40°C.

The temperature in the reaction step is not particularly limited, and is, for example, 0°C to 60°C, preferably room temperature to 45°C, and more preferably 40°C.

The pressure in the reaction step is, for example, 0.1 MPa to 1 MPa, preferably 0.1 MPa to 0.5 MPa, and more preferably 0.1 MPa.

The reaction time in the reaction step is not particularly limited, and is, for example, 0.5 hours to 24 hours.

The reaction step is preferably carried out under an inert gas atmosphere.

After the reaction step is completed, extraction may be conducted with an organic solvent, and the resultant organic layer may be subjected to a post-treatment such as drying and concentrating. Furthermore, if necessary, the resultant in the reaction step may be purified by an operation such as recrystallization or chromatography.

The present invention will be described more specifically below with reference to Examples. In addition, the present invention is not limited to the following examples.

### [Example 1]

Ethyl 3-[5-(2-chloro-3,3,3-trifluoroprop-1-en-1-yl)-1-methyl-1H-imidazol-2-yl]-2-(ethylthio)-3-oxopropanoate was obtained by the following procedure.

.

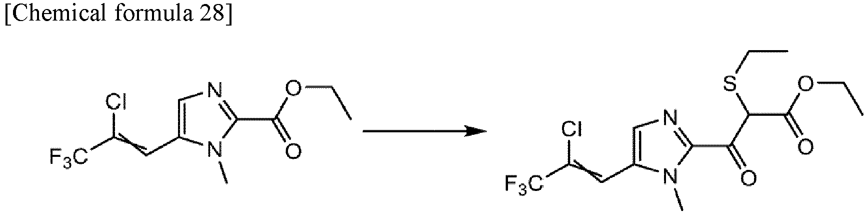

Chloroform (32 ml) was put in a 200 ml four-necked flask purged with nitrogen and then cooled to 0°C. Then, titanium tetrachloride (6.8 g) and isopropyl alcohol (2.1 g) were added dropwise while stirring. Stirring was continued at 0°C for 30 minutes. Then, ethyl 2-(ethylthio)acetate (5.5 g) was added dropwise while stirring at 0°C, followed by adding dropwise ethyl 5-(2-chloro-3,3,3-trifluoroprop-1-en-1-yl)-1-methyl-1H-imidazole-2-carboxylate (9.5 g) (E/Z: 14.5/85.5) while stirring at 0°C. Subsequently, stirring was continued at 0°C for 30 minutes. Then, triethylamine (10.8 g) was added dropwise while stirring at 0°C, and after completion of the dropwise addition, stirring was continued at room temperature for 24 hours. Then, the resultant was cooled to 0°C. 32 ml of chloroform was added thereto, and titanium tetrachloride (6.8 g) and isopropyl alcohol (2.1 g) were added dropwise while stirring. After completion of the dropwise addition, stirring was conducted at 0°C for 30 minutes. Subsequently, triethylamine (10.8 g) was added dropwise while stirring at 0°C, and stirring was continued at room temperature for four hours.

Chloroform (93 ml) and 13% hydrochloric acid (121 g) were added dropwise to the resultant liquid while stirring under ice-cooling, and stirring was continued for a while. Then, the resultant was subjected to a liquid-separation, and an organic layer (separated lower liquid layer) was concentrated under reduced pressure. The obtained concentrate was subjected to column-purification to obtain ethyl 3-[5-(2-chloro-3,3,3-trifluoroprop-1-en-1-yl)-1-methyl-1H-imidazol-2-yl]-2-(ethylthio)-3-oxopropanoate (5.40 g) (E/Z: 19.4/80.6) (purity 98.1 wt%, yield 44.5 mol%).

### [Reference Example 1]

1-[5-(2-Chloro-3,3,3-trifluoroprop-1-en-1-yl)-1-methyl-1H-imidazol-2-yl]-2-(ethylthio)-ethan-1-one was obtained by the following procedure.

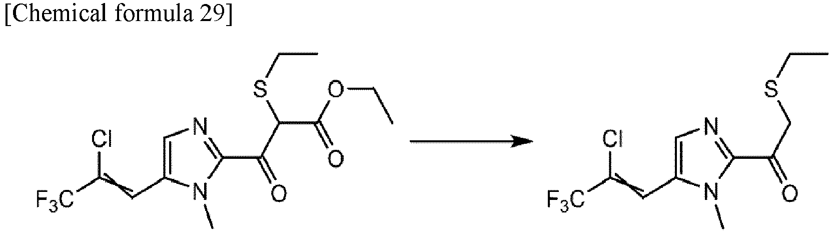

Ethyl 3-[5-(2-chloro-3,3,3-trifluoroprop-1-en-1-yl)-1-methyl-1H-imidazol-2-yl]-2-(ethylthio)-3-oxopropanoate (5.40 g) (E/Z: 19.4/80.6) and ethyl alcohol (25 ml) were put in a 200 ml four-necked flask, followed by adding dropwise 8.3 wt% sulfuric acid (18. 0 g) while stirring at room temperature. Then, the resultant was heated to 100°C and stirred for 14 hours.

The obtained liquid was cooled to 30°C, water (39 ml) was added thereto, a first extraction was performed with chloroform (39 ml), and then a second extraction was performed with chloroform (13 ml). The organic layers obtained by the extractions were combined, concentrated under reduced pressure and dried to obtain 1-[5-(2-chloro-3,3,3-trifluoroprop-1-en-1-yl)-1-methyl-1H-imidazol-2-yl]-2-(ethylthio)-ethan-1 -one (3.90 g) (E/Z: 24.5/75.5) (purity 95.1 wt%, yield 91.2 mol %).

### [Reference Example 2]

1-[5-(2-Chloro-3,3,3-trifluoroprop-1-en-1-yl)-1-methyl-1H-imidazol-2-yl]-2-(ethylsulfonyl)-ethan-1-one was obtained by the following procedure.

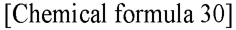

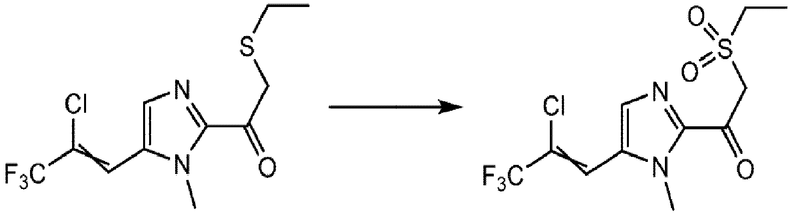

1-[5-(2-Chloro-3,3,3-trifluoroprop-1-en-1-yl)-1-methyl-1H-imidazol-2-yl]-2-(ethylthio)-ethan-1-one (3.90 g) (E/Z: 24.5/75.5), ethyl acetate (12 ml), and sodium tungstate dihydrate (0.063 g) were put in a 100 ml four-necked flask, and then cooled to 0°C. A 30% hydrogen peroxide solution (3.0 g) was added dropwise thereto while stirring. Then, the resultant was warmed to room temperature and stirred for 3 hours.

A 6.4% aqueous sodium hydrogen sulfite solution (27 g) was added to the obtained liquid, and a first extraction was performed with ethyl acetate (25 ml), a second extraction was performed with ethyl acetate (15 ml), and then a third extraction was performed with ethyl acetate (10 ml). The organic layers obtained by the extractions were combined, concentrated under reduced pressure and dried. The dried product was subjected to a crystallization treatment using a solvent obtained by mixing n-hexane and ethyl acetate at a volume ratio of 8:2 (n-hexane:ethyl acetate), thereby obtaining 1-[5-(2-chloro-3,3,3 -trifluoroprop-1-en-1-yl)-1-methyl-1H-imidazol-2-yl]-2-(ethylsulfonyl)-ethan-1-one (2.67 g) (E/Z: 12 .4/87.6) (purity 97.9 wt%, yield 64.0 mol%).

In addition, the filtrate obtained by the crystallization treatment was concentrated under reduced pressure and dried. The dried product was subjected to column-purification to collect secondary crystals (0.7 g) (E/Z: 45.7/54.3) (purity 93.7 wt%, yield 16.1 mol%).

### [Example 2]

### Production of ethyl (Z)-5-(2-chloro-3,3,3-trifluoroprop-1-en-1-yl)-1-methyl-1H-imidazole-2-carboxylate

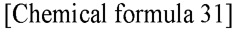

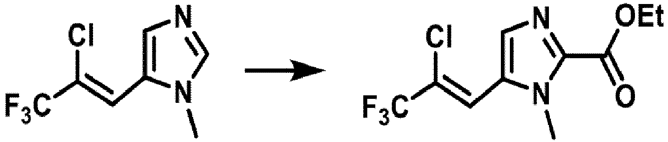

1.413 g of (Z)-5-(2-chloro-3,3,3-trifluoroprop-1-en-1-yl)-1-methyl-1H-imidazole (purity 96.0%, 5.00 mmol) and 0.904 g (7.00 mmol) of diisopropylethylamine were dissolved in 5.0 mL of chloroform. The liquid mixture was heated to 40°C in a water bath, 0.760 g (7.00 mmol) of ethyl chloroformate was added dropwise while stirring, and then stirring was continued at the same temperature for 2.0 hours. After confirming the disappearance of the raw materials, quantitative analysis of the reaction liquid was conducted, thereby revealing that the yield of ethyl (Z)-5-(2-chloro-3,3,3-trifluoroprop-1-en-1-yl)-1-methyl-1H-imidazole-2-carboxylate was 95.2%.

### [Example 3]

### Production of ethyl (Z)-5-(2-chloro-3,3,3-trifluoroprop-1-en-1-yl)-1-methyl-1H-imidazole-2-carboxylate

1.413 g of (Z)-5-(2-chloro-3,3,3-trifluoroprop-1-en-1-yl)-1-methyl-1H-imidazole (purity 96.0%, 5.00 mmol), and 1.422 g of diisopropylethylamine (11.00 mmol) were dissolved in 5.0 mL of chloroform. This liquid mixture was added dropwise to a solution of 1.194 g (11.00 mmol) of ethyl chloroformate in 5.0 mL of chloroform while stirring at 40°C, and then stirring was continued at the same temperature for 2.0 hours. After terminating the reaction, quantitative analysis of the reaction liquid was conducted, thereby revealing that the yield of ethyl (Z)-5-(2-chloro-3,3,3-trifluoroprop-1-en-1-yl)-1-methyl-1H-imidazole-2-carboxylate was 95.7%.

Next, an example of production using triethylamine instead of diisopropylethylamine is described below.

### [Reference Example 3]

### Production of ethyl (Z)-5-(2-chloro-3,3,3-trifluoroprop-1-en-1-yl)-1-methyl-1H-imidazole-2-carboxylate

1.000 g of (Z)-5-(2-chloro-3,3,3-trifluoroprop-1-en-1-yl)-1-methyl-1H-imidazole (purity 96.0%, 4.70 mmol), and 1.057 g (10.40 mmol) of triethylamine were dissolved in 9.5 mL of chloroform. The liquid mixture was added dropwise to a solution of 1.134 g (10.40 mmol) of ethyl chloroformate in 7.0 mL of chloroform while stirring at 0°C, and then stirring was continued at room temperature for 6.0 hours. After terminating the reaction, quantitative analysis of the reaction liquid was conducted, thereby revealing that the yield of ethyl (Z)-5-(2-chloro-3,3,3-trifluoroprop-1-en-1-yl)-1-methyl-1H-imidazole-2-carboxylate was 39.3%.

### [Industrial Applicability]

The production method of the present invention makes it possible to efficiently obtain a 2-alkylthio-1-imidazoyl ethanone compound, which is a main partial structure that constitutes a compound which serves as a raw material of a pharmaceutical or agricultural chemical.

## Claims

1. A method of producing a compound of formula (3), comprising:
chemically reacting a compound of formula (1) and a compound of formula (2) in a presence of a Lewis acid and a base, 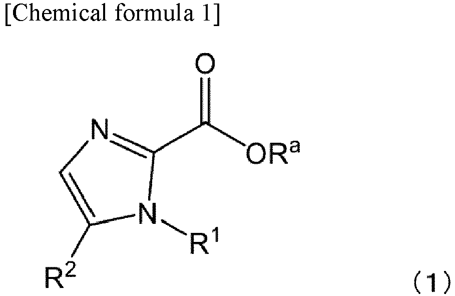
(in the formula (1), R¹ is a C1-6 alkyl group, R² is a hydrogen atom, a halogeno group, a substituted or unsubstituted C1-6 alkyl group, a substituted or unsubstituted C2-6 alkenyl group, or a 1,3-dioxolan-2-yl group, and R^{a} is a C1-6 alkyl group), 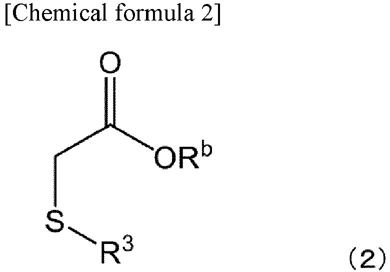
(in the formula (2), R³ is a C1-6 alkyl group, and R^{b} is a C1-6 alkyl group), 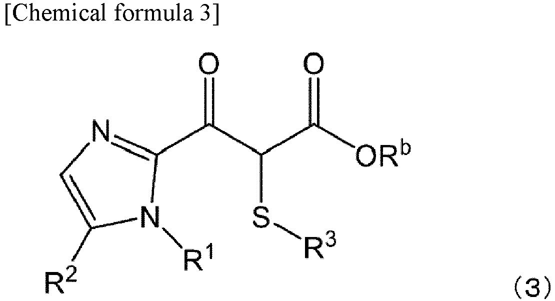
(in the formula (3), R¹, R², R³, and R^{b} are identical to R¹, R², R³, and R^{b} in the formula (1) and the formula (2), respectively).

2. The production method according to claim 1, wherein the Lewis acid is a Ti compound of formula (4):
[Chemical formula 4]
Ti(X)ₘ(OR^{c})ₙ (4)
(in the formula (4), X is a halogeno group, R^{c} is a C1-6 alkyl group, m is an integer of 0 to 4, n is an integer of 0 to 4, and m+n=4).

3. A compound of formula (3a): 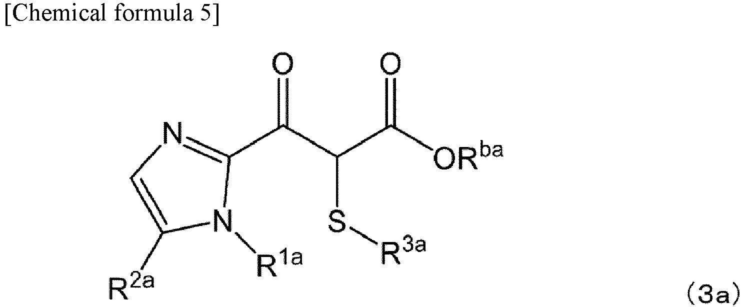 (in the formula (3a), R^{1a} is a C1-6 alkyl group, R^{2a} is a C2-6 haloalkenyl group, R^{3a} is a C1-6 alkyl group, and R^{ba} is a C1-6 alkyl group).

4. A compound of formula (1a): 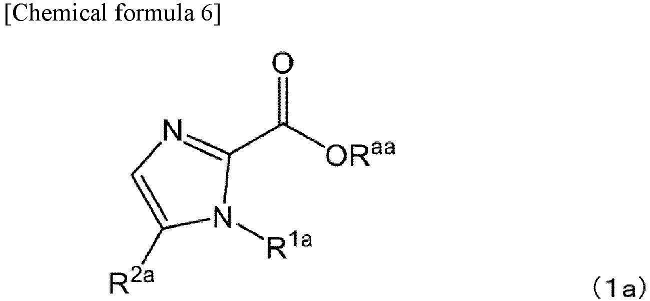 (in the formula (1a), R^{1a} is a C1-6 alkyl group, R^{2a} is a C2-6 haloalkenyl group, and R^{aa} is a C1-6 alkyl group).

5. A method of producing a compound of formula (1a), comprising:
an addition step in which a chloroformate of formula: ClCO₂R^{aa} (in the formula, R^{aa} is a C1-6 alkyl group) is added to a liquid mixture of a compound of formula (7), diisopropylethylamine and chloroform; and
a reaction step in which the compound of formula (7) and the chloroformate are reacted in a presence of the diisopropylethylamine to obtain the compound of formula (1a), 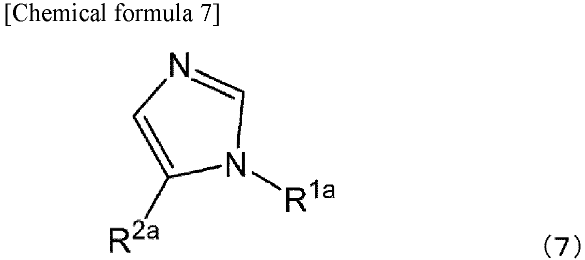
(in the formula (7), R^{1a} is a C1-6 alkyl group, and R^{2a} is a C2-6 haloalkenyl group) 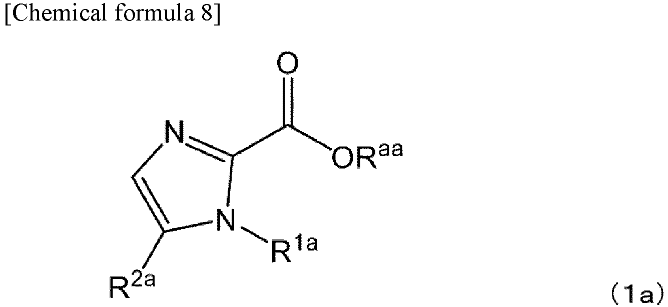
(in the formula (1a), R^{1a} is a C1-6 alkyl group, R^{2a} is a C2-6 haloalkenyl group, and R^{aa} is a C1-6 alkyl group).
